# EUROPÄISCHE PATENTSCHRIFT

(11) **EP 2 288 606 B1**
(45) Veröffentlichungstag und Bekanntmachung des Hinweises auf die Patenterteilung: **15.02.2012**
(21) Anmeldenummer: 09761414.3
(22) Anmeldetag: 03.06.2009
(51) Int. Cl.: C07D 413/14, A61K 31/5377, A61P 7/02

(54) **NEUE COKRISTALL-VERBINDUNG VON RIVAROXABAN UND MALONSÄURE**
NEW CO-CRYSTAL COMPOUND OF RIVAROXABAN AND MALONIC ACID
NOUVEAU COMPOSÉ DE RIVAROXABAN ET D ACIDE MALONIQUE CONTENANT DES CO-CRISTAUX

(30) Priorität: 12.06.2008 DE 102008028071
(43) Veröffentlichungstag der Anmeldung: 02.03.2011
(73) Patentinhaber: Bayer Pharma Aktiengesellschaft, 13353 Berlin (DE)
(72) Erfinder: GRUNENBERG, Alfons, 41539 Dormagen (DE); FÄHNRICH, Karsten, 79639 Grenzach-Whylen (DE); QUECKENBERG, Olaf, 51467 Bergisch Gladbach (DE); REUTE, Christiane, 51375 Leverkusen (DE); KEIL, Birgit, 40231 Düsseldorf (DE); GUSHURST, Karen, Sue, West Lafayette In 47906 (US); STILL, Ezra, John, West Lafayette IN 47906 (US)
(86) Internationale Anmeldenummer: PCT/EP2009/003952
(87) Internationale Veröffentlichungsnummer: WO 2009/149851

(56) Entgegenhaltungen:
- WO-A-01/47919
- WO-A-2005/068456
- WO-A-2007/039132

## Beschreibung

Die vorliegende Erfindung betrifft eine neue Cokristall-Verbindung von 5-Chlor-N-({(5*S*)-2-oxo-3-[4-(3-oxo-4-morpholinyl)-phenyl]-1,3-oxazolidin-5-yl}-methyl)-2-thiophencarboxamid (Rivaroxaban) und Malonsäure, Verfahren zu deren Herstellung, diese enthaltende Arzneimittel sowie deren Verwendung bei der Bekämpfung von Krankheiten.

Die Verbindung 5-Chlor-N-({(5*S*)-2-oxo-3-[4-(3-oxo-4-morpholinyl)-phenyl]-1,3-oxazolidin-5-yl}-methyl)-2-thiophencarboxamid (Rivaroxaban) ist aus WO 01/47919 bekannt und entspricht der Formel (1):

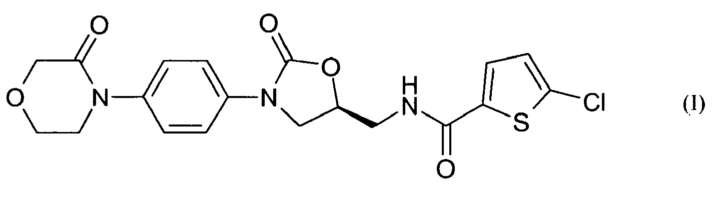

Die Verbindung der Formel (I) ist ein niedermolekularer, oral applizierbarer Inhibitor des Blutgerinnungsfaktors Xa, der zur Prophylaxe, Sekundärprophylaxe und/oder Behandlung von verschiedenen thromboembolischen Erkrankungen eingesetzt werden kann (siehe hierzu WO 01/47919), insbesondere von Herzinfarkt, Angina Pectoris (eingeschlossen instabile Angina), Reokklusionen und Restenosen nach einer Angioplastie oder aortokoronarem Bypass, Hirnschlag, transitorischen ischämischen Attacken, peripheren arteriellen Verschlusskrankheiten, Lungenembolien oder tiefen venösen Thrombosen.

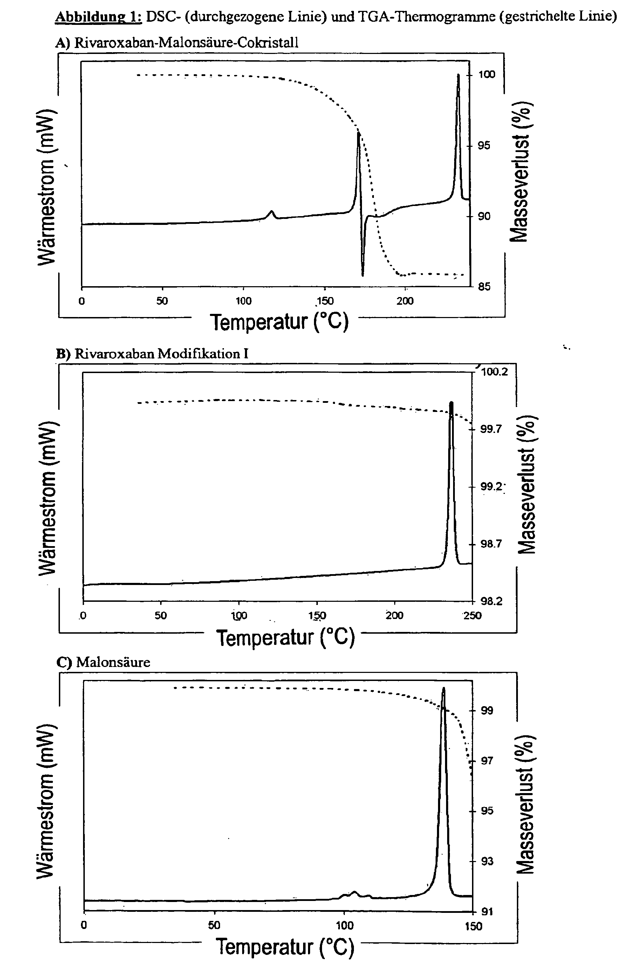

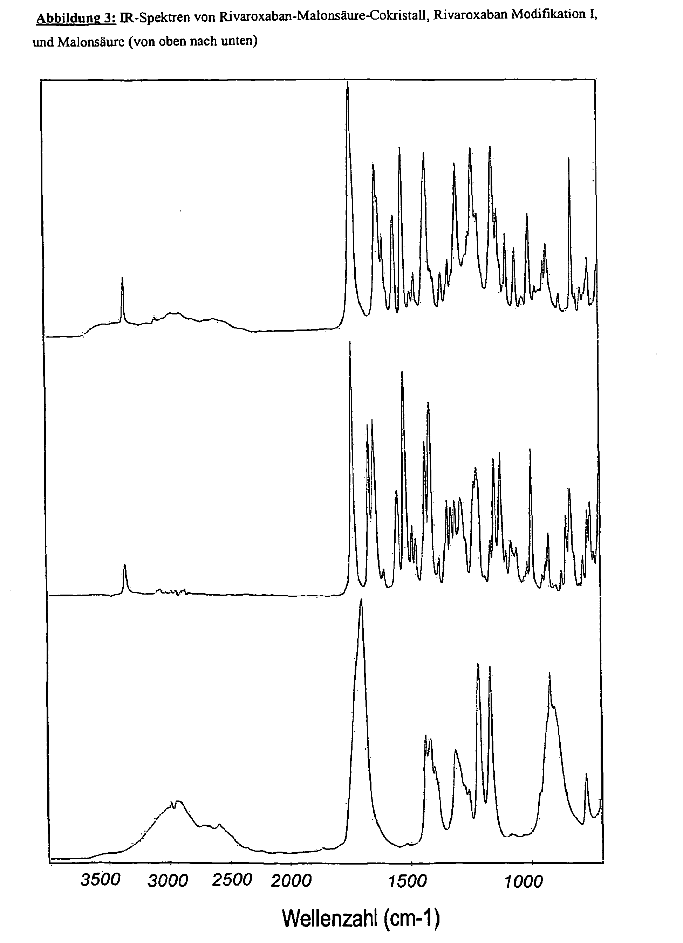

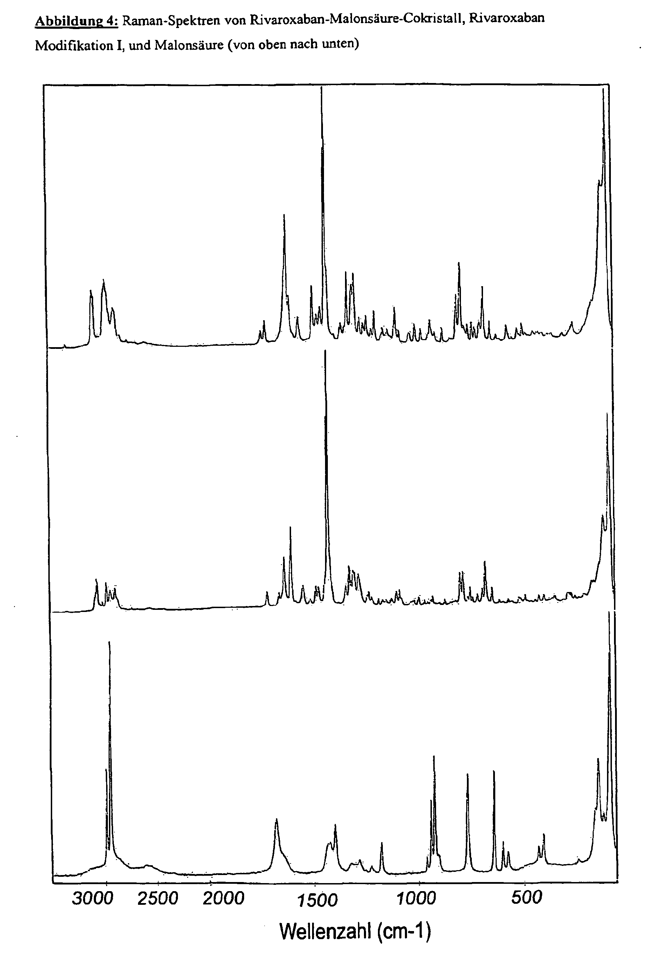

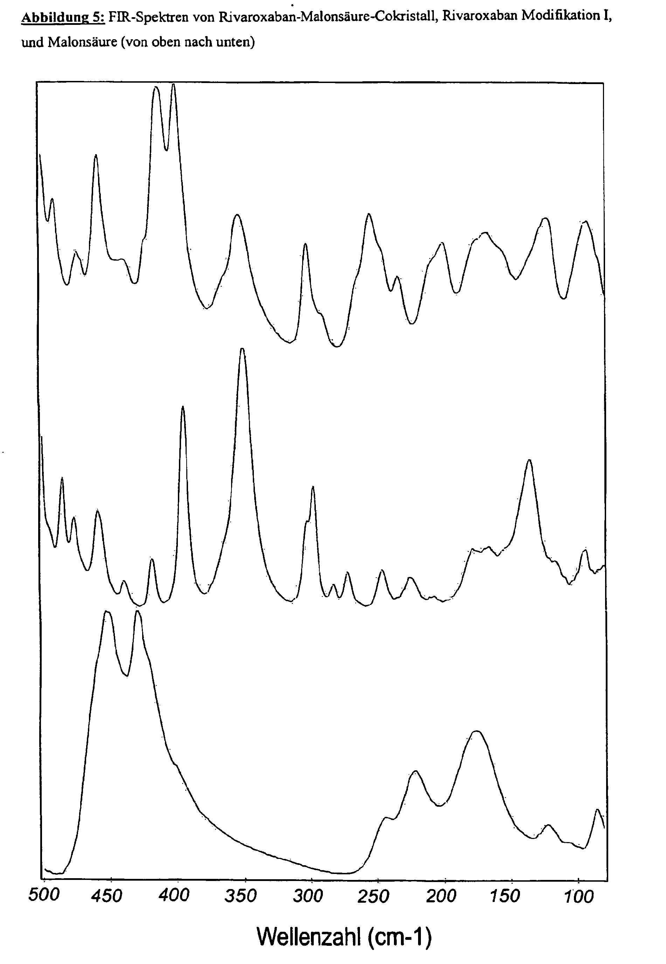

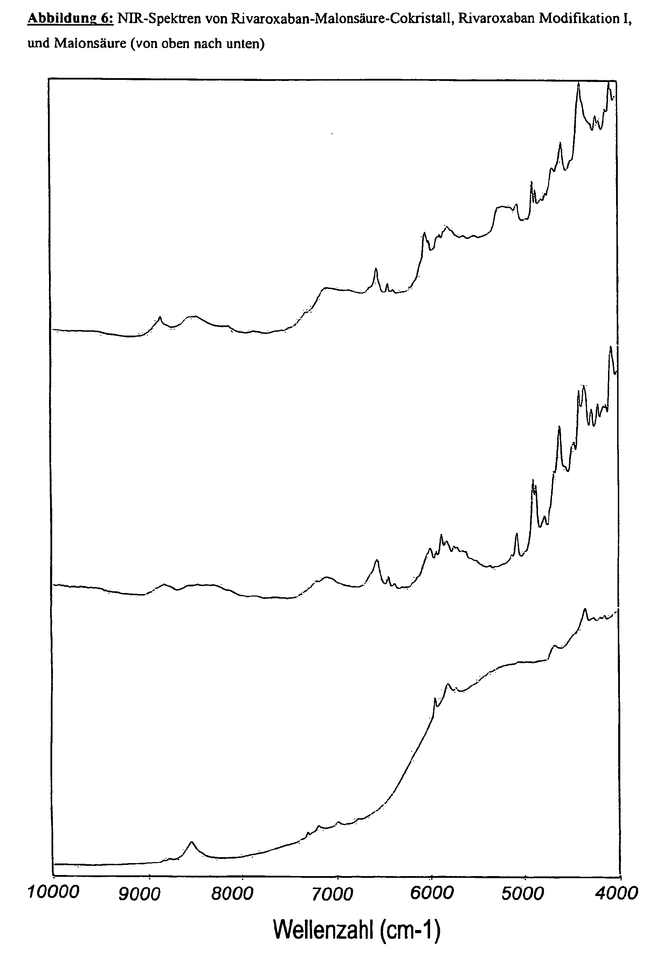

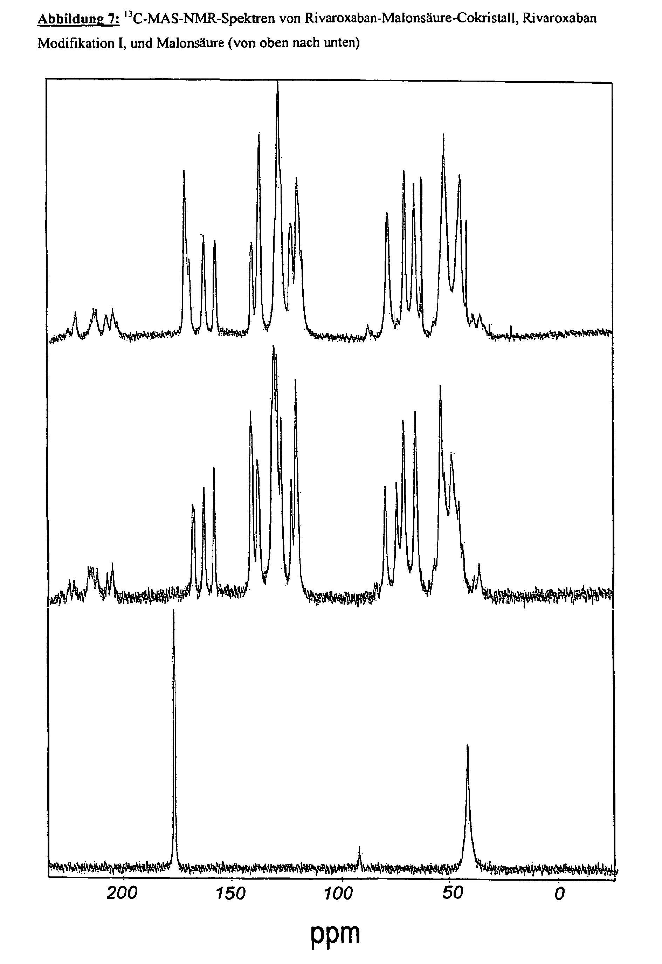

Die Verbindung der Formel (I) lässt sich, wie in WO 01/47919 beschrieben, herstellen. Dabei wird die Verbindung der Formel (I) in einer Kristallmodifikation erhalten, die im Folgenden als Modifikation I bezeichnet wird. Modifikation I hat einen Schmelzpunkt von 230°C und ein charakteristisches Röntgendiffraktogramm, IR-Spektrum, Raman-Spektrum, FIR-Spektrum, NIR-Spektrum und ¹³C-Festkörper-NMR-Spektrum (Tabelle 1-7, Abbildung 1-7).

Gegenstand der vorliegenden Erfindung ist die Verbindung Rivaroxaban-Malonsäure-Cokristall der Formel (II)

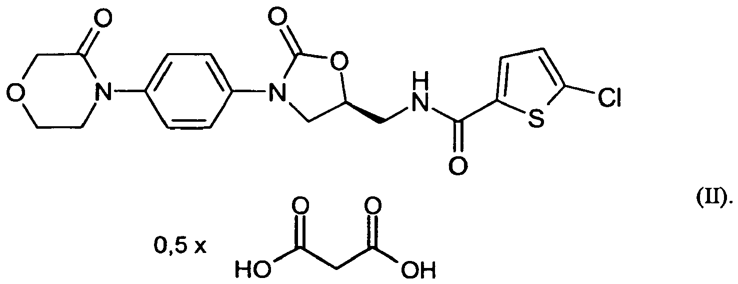

Überraschenderweise weist Rivaroxaban-Malonsäure-Cokristall der Formel (II) eine um den Faktor 2,5 höhere Löslichkeit auf als die Verbindung der Formel (I) in der Modifikation I.

Durch den erfindungsgemäßen Einsatz der Verbindung als Rivaroxaban-Malonsäure-Cokristall der Formel (II) wird sichergestellt, dass eine im Vergleich zur bekannten Verbindung der Formel (I) in der Modifikation I 2,5-fach höhere Löslichkeit erreicht wird.
Abbildung 1: DSC- (durchgezogene Linie) und TGA-Thermogramme (gestrichelte Linie)
   A) Rivaroxaban-Malonsäure-Cokristall
   B) Rivaroxaban Modifikation I
   C) Malonsäure
Abbildung 2: Röntgendiffraktogramme von Rivaroxaban-Malonsäure-Cokristall, Rivaroxaban Modifikation I, und Malonsäure (von oben nach unten)
Abbildung 3: IR-Spektren von Rivaroxaban-Malonsäure-Cokristall, Rivaroxaban Modifikation I, und Malonsäure (von oben nach unten)
Abbildung 4: Raman-Spektren von Rivaroxaban-Malonsäure-Cokristall, Rivaroxaban Modifikation I, und Malonsäure (von oben nach unten)
Abbildung 5: FIR-Spektren von Rivaroxaban-Malonsäure-Cokristall, Rivaroxaban Modifikation I, und Malonsäure (von oben nach unten)
Abbildung 6: NIR-Spektren von Rivaroxaban-Malonsäure-Cokristall, Rivaroxaban Modifikation I, und Malonsäure (von oben nach unten)
Abbildung 7: ¹³C-MAS-NMR-Spektren von Rivaroxaban-Malonsäure-Cokristall, Rivaroxaban Modifikation I, und Malonsäure (von oben nach unten)

Cokristall: Ein Cokristall ist ein Mischkristall, der aus zwei verschiedenen Komponenten besteht (A. I. Kitaigorodskii, Mixed crystals, Springer-Verlag, New York, 1984), die bei Raumtemperatur fest sind (C. B Aakeröy, D. J. Salmon, Building cocrystals with molecular sense and supramolecular sensibility, Cryst. Eng. Comm. 7 (2005) 439-448).

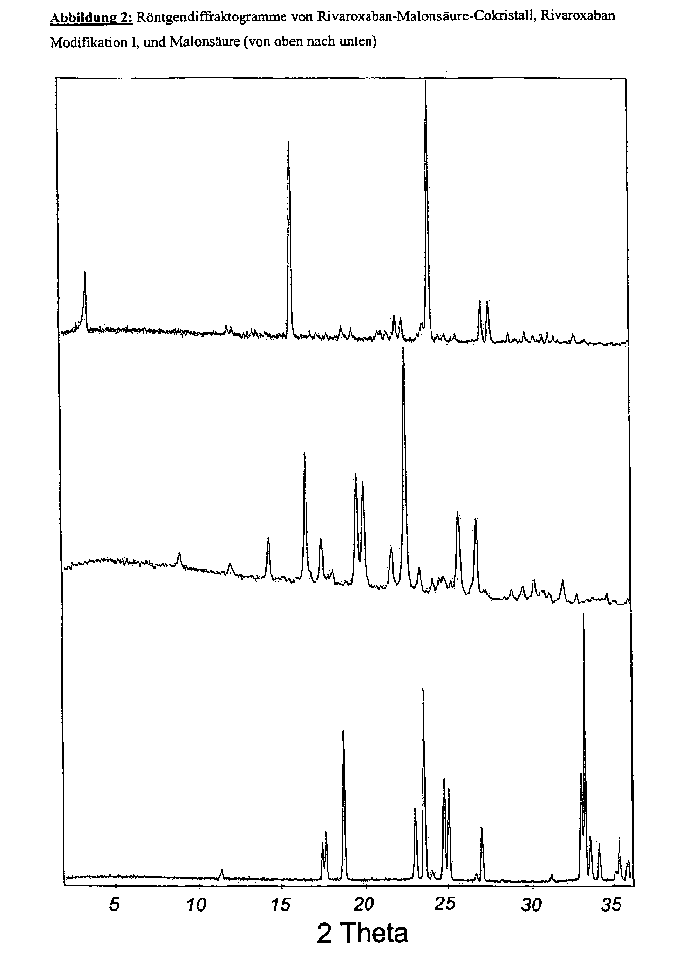

Rivaroxaban-Malonsäure-Cokristall der Formel (II) hat im Vergleich zur Verbindung der Formel (I) in der Modifikation I und Malonsäure der Formel (III) ein klar unterscheidbares Röntgendiffraktogramm, IR-Spektrum, NIR-Spektrum, FIR-Spektrum, Raman-Spektrum und ¹³C-Festkörper-NMR-Spektrum (Abbildung 2-7). Die Verbindung der Formel (II) schmilzt bei 160°C bzw. wandelt sich bei etwa 115°C um und ist damit klar unterscheidbar von der Verbindung der Formel (I) in der Modifikation I (Schmelzpunkt 230°C) und Malonsäure der Formel (III) (Schmelzpunkt etwa 135°C bzw.Umwandlungspunkt etwa 85-110°C). Im Gegensatz zur Verbindung der Formel (I) in der Modifikation I hat die Verbindung der Formel (II) einen Masseverlust von 14%.

Malonsäure, Verbindung der Formel (III)

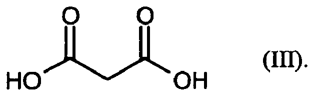

Eine Einkristallstrukturanalyse bestätigt, dass Rivaroxaban-Malonäure-Cokristall im molaren Verhältnis von 2:1 vorliegt (Tabelle 8).

Weiterer Gegenstand der vorliegenden Erfindung ist die Verbindung Rivaroxaban-Malonsäure-Cokristall der Formel (II), die im Röntgendiffraktogramm einen Reflex bei einem 2-Theta-Winkel von 15,8 aufweist.

Weiterer Gegenstand der vorliegenden Erfindung ist die Verbindung Rivaroxaban-Malonsäure-Cokristall der Formel (II), die im Raman-Spektrum einen Peak bei 1617 cm⁻¹ aufweist.

Die erfindungsgemäße Verbindung der Formel (II) wird in pharmazeutischen Formulierungen in hoher Reinheit eingesetzt. Aus Stabilitätsgründen enthält eine pharmazeutische Formulierung hauptsächlich die Verbindung der Formel (II) und keine größeren Anteile einer anderen Form wie beispielsweise einer anderen Modifikation oder eines Solvates der Verbindung der Formel (II). Bevorzugt enthält das Arzneimittel mehr als 90 Gewichtsprozente, besonders bevorzugt mehr als 95 Gewichtsprozente der Verbindung der Formel (II) bezogen auf die Gesamtmenge der enthaltenen Verbindung der Formel (II).

Weiterer Gegenstand der vorliegenden Erfindung ist der Einsatz der Verbindung der Formel (II) zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die erfindungsgemäße Verbindung eignet sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus ist die erfindungsgemäße Verbindung zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommt die erfindungsgemäße Verbindung auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem kann die erfindungsgemäße Verbindung zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäße Verbindung kann darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindung zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindung zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend die erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- sowie Fibrinogen-Rezeptor-Antagonisten (Glycoprotein-IIb/IIIa-Antagonisten).

Weiterer Gegenstand der vorliegenden Erfindung sind Arzneimittel, die die erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäße Verbindung kann systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege kann die erfindungsgemäße Verbindung in geeigneten Applikationsformen verabreicht werden.

Für die orale Applikation eignen sich nach dem Stand der Technik funktionierende, die erfindungsgemäß Verbindung schnell und/oder modifiziert abgebende Applikationsformen, die die Verbindung der Formel (II) enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Suspensionen oder Aerosole.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Suspensionen, Lyophilisaten oder sterilen Pulvern.

Für die sonstigen Applikationswege eignen sich z.B. Inhalationsarzneiformen (u.a. Pulverinhalatoren, Nebulizer), lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäße Verbindung kann in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Weiterer Gegenstand der vorliegenden Erfindung ist ein Verfahren zur Herstellung der Verbindung Rivaroxaban-Malonsäure-Cokristall der Formel (II), indem der Wirkstoff Rivaroxaban der Formel (I) in der Modifikation I und das Reagenz Malonsäure der Formel (III) in einem inerten Lösungsmittel bei einer Temperatur zwischen 50°C und 90°C gelöst und das Lösungsmittel abgedampft wird.

Weiterer Gegenstand der vorliegenden Erfindung ist ein Verfahren zur Herstellung der Verbindung Rivaroxaban-Malonsäure-Cokristall der Formel (II), indem der Wirkstoff Rivaroxaban der Formel (I) in der Modifikation I und das Reagenz Malonsäure der Formel (III) in einem inerten Lösungsmittel im molaren Verhältnis 1:1 bei einer Temperatur zwischen 50°C und 90°C gelöst werden. Das Lösemittel wird vorzugsweise zwischen 20°C und 25°C verdunstet. Man erhält so die Verbindung Rivaroxaban-Malonsäure-Cokristall der Formel (II).

Weiterer Gegenstand der vorliegenden Erfindung ist ein Verfahren zur Herstellung der Verbindung Rivaroxaban-Malonsäure-Cokristall der Formel (II), indem der Wirkstoff Rivaroxaban der Formel (I) in der Modifikation I in einem inerten Lösemittel unter Rühren und Erhitzen, vorzugsweise zwischen 50°C und 90°C gelöst und der heißen Lösung 1,5 eq Malonsäure der Formel (III) zugesetzt werden. Das Volumen der Lösung wird vorzugsweise zwischen 50°C und 90°C auf ca. die Hälfte reduziert, in ein anderes Gefäß überführt und verschlossen. Die Lösung wird 4 h bei einer Temperatur zwischen 20°C und 25°C abgekühlt. Die entstandenen Kristalle werden isoliert und getrocknet. Man erhält so die Verbindung Rivaroxaban-Malonsäure-Cokristall der Formel (II).

Weiterer Gegenstand der vorliegenden Erfindung ist ein Verfahren zur Herstellung der Verbindung Rivaroxaban-Malonsäure-Cokristall der Formel (II), indem der Wirkstoff Rivaroxaban der Formel (I) in der Modifikation I und das Reagenz Malonsäure der Formel (III) in einem inerten Lösungsmittel im molaren Verhältnis 1:1 bei einer Temperatur zwischen 50°C und 90°C gelöst werden. Das Volumen wird bevorzugt zwischen 50°C und 90°C auf ca. die Hälfte reduziert. Die heiße Lösung wird mit Kristallen der Verbindung Rivaroxaban-Malonsäure-Cokristall der Formel (II) angeimpft. Die heiße Lösung wird innerhalb von ca.15 Minuten vorzugsweise auf zwischen 20°C bis 25°C abgekühlt. Der Niederschlag wird isoliert und getrocknet. Man erhält so die Verbindung Rivaroxaban-Malonsäure-Cokristall der Formel (II).

Weiterer Gegenstand der vorliegenden Erfindung ist ein Verfahren zur Herstellung der Verbindung Rivaroxaban-Malonsäure-Cokristall der Formel (II), indem eine gesättigte Lösung des Reagenz Malonsäure der Formel (III) in einem inerten Lösemittel hergestellt wird, in der vorzugsweise zwischen 50°C und 90°C der Wirkstoff Rivaroxaban der Formel (I) der Modifikation I gelöst wird. Die Lösung wird langsam bevorzugt auf 20°C bis 25°C abgekühlt. Nach ca. 2 h wird der Niederschlag abgesaugt und getrocknet. Man erhält so die Verbindung Rivaroxaban-Malonsäure-Cokristall der Formel (II).

Weiterer Gegenstand der vorliegenden Erfindung ist ein Verfahren zur Herstellung der Verbindung Rivaroxaban-Malonsäure-Cokristall der Formel (II), indem der Wirkstoff Rivaroxaban der Formel (I) in der Modifikation I und das Reagenz Malonsäure der Formel (III) in einem inerten Lösungsmittel bei einer Temperatur zwischen 20°C und 25°C suspendiert werden und der Niederschlag isoliert wird.

Weiterer Gegenstand der vorliegenden Erfindung ist ein Verfahren zur Herstellung der Verbindung Rivaroxaban-Malonsäure-Cokristall der Formel (II), indem eine Lösung des Reagenz Malonsäure der Formel (III) in einem inerten Lösemittel hergestellt wird, in der vorzugsweise zwischen 20°C und 25°C der Wirkstoff Rivaroxaban der Formel (I) in der Modifikation I suspendiert wird. Die Suspension wird mindestens 48 h vorzugsweise zwischen 20°C und 25°C verschlossen gerührt. Der Niederschlag wird isoliert und getrocknet. Man erhält so die Verbindung Rivaroxaban-Malonsäure-Cokristall der Formel (II).

Als inerte Lösungsmittel eignen sich niedere Alkohole wie beispielsweise Ethanol, 2-Propanol oder 2,2,2-Trifluorethanol, oder Ketone wie beispielsweise Aceton, oder andere Lösungsmittel wie beispielsweise Acetonitril. Bevorzugt ist 2,2,2-Trifluorethanol oder Aceton.

Bevorzugt wird die Verbindung Rivaroxaban-Malonsäure-Cokristall der Formel (II) hergestellt, indem Malonsäure der Formel (III) und der Wirkstoff Rivaroxaban der Formel (I) der Modifikation I im molaren Verhältnis 1:1 in 2,2,2-Trifluorethanol vorzugsweise zwischen 50°C und 90°C gelöst werden. Das Volumen der Lösung wird auf ca. die Hälfte reduziert. Der durch Animpfen der heißen Lösung mit Kristallen von Rivaroxaban-Malonsäure-Cokristall der Formel (II) erhaltene Niederschlag wird isoliert und getrocknet. Man erhält so die Verbindung Rivaroxaban-Malonsäure-Cokristall der Formel (II).

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Ausführungsbeispiele

Die Thermogramme wurden unter Verwendung eines Differential Scanning Calorimeters Diamond DSC bzw. Pyris-1 und Thermogravimetric Analyzer Pyris-1 der Fa. Perkin-Elmer erhalten. Die Röntgendiffraktogramme wurden in einem Stoe-Transmissionsdiffraktometer registriert. Die ¹³C-MAS-NMR-Spektren wurden mit einem Kernresonanzspektrometer DMX 300 der Fa. Bruker aufgenommen. Die IR-, FIR-, NIR- und Raman-Spektren wurden mit Fourier-Spektrometern Tensor 27/Miracle ATR (IR), IFS 66v (FIR), Vector 22/N (NIR) und RFS 100 (Raman) der Fa. Bruker aufgenommen.

### Beispiel 1: Rivaroxaban der Formel (I) in der Modifikation I

Die Herstellung der Modifikation I der Titelverbindung ist in WO 01/47919 beschrieben.

### Beispiel 2: Herstellung von Rivaroxaban-Malonsäure-Cokristall

### Beispiel 2.1

27 mg Rivaroxaban der Formel (I) in der Modifikation I wurden in 3 mL 2,2,2-Trifluorethanol gelöst und die Lösung über einen 0,2 µm Spritzenfilter filtriert. Die Lösung wurde mit 0,2 mL Malonsäurelösung [75 mg Malonsäure in 2 mL Methanol] versetzt. Die Lösung wurde bei Raumtemperatur verdunstet und die entstandenen Kristalle getrocknet. Die Kristalle wurden Raman-spektroskopisch geprüft und entsprechen der Titelverbindung in der Formel (II).

### Beispiel 2.2

87 mg Rivaroxaban der Formel (I) in der Modifikation I wurden in 0,45 mL 2,2,2-Trifluorethanol unter Rühren und Erhitzen zwischen 70°C bis 80°C gelöst. 30 mg Malonsäure der Formel (III) wurden der heißen Lösung zugesetzt. Das Volumen der Lösung wurde durch offenes Erhitzen auf ca. 0,2 mL reduziert und die heiße Lösung anschließend in ein anderes Glasgefäß überführt. Das Gefäß wurde verschlossen und bei Raumtemperatur abgekühlt. Nach 4 h wurde der entstandene Niederschlag abgesaugt. Die Kristalle wurden an der Luft zwischen 20°C und 25°C getrocknet. Die Kristalle wurden Raman-spektroskopisch geprüft und entsprechen der Titelverbindung in der Formel (II).

### Beispiel 2.3

87 mg Rivaroxaban der Formel (I) in der Modifikation I und 21 mg Malonsäure der Formel (III) wurden in 0,5 mL 2,2,2-Trifluorethanol unter Rühren und Erhitzen zwischen 70°C bis 80°C gelöst. Das Volumen der Lösung wurde durch offenes Erhitzen auf ca. 0,15 mL reduziert und die heiße Lösung mit Kristallen Rivaroxaban-Malonsäure-Cokristall der Formel (II) angeimpft. Die Lösung wurde innerhalb von 15-20 Minuten auf Raumtemperatur abgekühlt. Der entstandene Niederschlag wurde abgesaugt und an der Luft zwischen 20°C und 25°C getrocknet. Der Niederschlag wurde Raman-spektroskopisch geprüft und entspricht der Titelverbindung in der Formel (II).

### Beispiel 2.4

871 mg Rivaroxaban der Formel (I) in der Modifikation I und 208 mg Malonsäure der Formel (III) wurden in 5 ml 2,2,2-Trifluorethanol suspendiert und auf 70°C bis 80°C erhitzt und gelöst. Die Lösung wurde durch Erhitzen zwischen 70°C und 80°C auf ca. 2,5 mL eingedampft. Die heiße Lösung wurde mit Kristallen Rivaroxaban-Malonsäure-Cokristall der Formel (II) angeimpft und innerhalb von 20 Minuten auf Raumtemperatur abgekühlt. Der entstandene Niederschlag wurde abgesaugt und an der Luft zwischen 20°C und 25°C getrocknet. Der Niederschlag wurde Raman-spektroskopisch geprüft und entspricht der Titelverbindung in der Formel (II).

### Beispiel 2.5

17 g Rivaroxaban der Formel (I) in der Modifikation I und 4 g Malonsäure der Formel (III) wurden in 100 ml 2,2,2-Trifluorethanol suspendiert und auf 50°C bis 90°C erhitzt und gelöst. Die Lösung wurde durch Erhitzen zwischen 50°C und 90°C auf ca. 50 mL eingedampft. Die heiße Lösung wurde mit Kristallen Rivaroxaban-Malonsäure-Cokristall der Formel (II) angeimpft und innerhalb von 20 Minuten auf Raumtemperatur abgekühlt. Der entstandene Niederschlag wurde abgesaugt und an der Luft zwischen 20°C und 25°C getrocknet. Der Niederschlag wurde Raman-spektroskopisch geprüft und entspricht der Titelverbindung in der Formel (II).

### Beispiel 2.6

800 mg Malonsäure der Formel (III) wurden in 3 mL Aceton gelöst und die Lösung über Nacht stehen gelassen. Zu 2 mL dieser Lösung wurden 33 mg Rivaroxaban der Formel (I) in der Modifikation I zugesetzt und suspendiert. Die Suspension wurde 48 h bei Raumtemperatur verschlossen gerührt. Die Kristalle wurden abgesaugt und an der Luft zwischen 20°C und 25°C getrocknet. Die Kristalle wurden Raman-spektroskopisch geprüft und entsprechen der Titelverbindung in der Formel (II).

### Beispiel 2.7

Es wurde eine gesättigte Lösung von Malonsäure der Formel (III) in Aceton hergestellt. Die Lösung wurde 12 h zwischen 20°C und 25°C gerührt und der ungelöste Rückstand über Faltenfilter abfiltriert. 103 mg Rivaroxaban der Formel (I) in der Modifikation I wurden mit 3 mL der gesättigten Malonsäurelösung versetzt und unter Rückfluss zwischen 50°C und 90°C darin gelöst. Die Lösung wurde innerhalb von 2 h auf Raumtemperatur abgekühlt. Der entstandene Niederschlag wurde abgesaugt und an der Luft zwischen 20°C und 25°C getrocknet. Die Kristalle wurden Raman-spektroskopisch geprüft und entsprechen der Titelverbindung in der Formel (II).

### Beispiel 2.8

Es wurde eine gesättigte Lösung von Malonsäure der Formel (III) in Ethanol hergestellt. Die Lösung wurde 12 h zwischen 20°C und 25°C gerührt und der ungelöste Rückstand über Faltenfilter abfiltriert. 103 mg Rivaroxaban der Formel (I) in der Modifikation I wurden mit 3 mL der gesättigten Malonsäurelösung versetzt und unter Rückfluss zwischen 50°C und 90°C darin gelöst. Die Lösung wurde innerhalb von 2 h auf Raumtemperatur abgekühlt. Der entstandene Niederschlag wurde abgesaugt und an der Luft zwischen 20°C und 25°C getrocknet. Der Niederschlag wurde Raman-spektroskopisch geprüft und entspricht der Titelverbindung in der Formel (II).

### Beispiel 2.9

Es wurde eine gesättigte Lösung von Malonsäure der Formel (III) in 2-Propanol hergestellt. Die Lösung wurde 12 h zwischen 20°C und 25°C gerührt und der ungelöste Rückstand über Faltenfilter abfiltriert. 103 mg Rivaroxaban der Formel (I) in der Modifikation I wurden mit 3 mL der gesättigten Malonsäurelösung versetzt und unter Rückfluss zwischen 50°C und 90°C darin gelöst. Die Lösung wurde innerhalb von 2 h auf Raumtemperatur abgekühlt. Der entstandene Niederschlag wurde abgesaugt und an der Luft zwischen 20°C und 25°C getrocknet. Der Niederschlag wurde Raman-spektroskopisch geprüft und entspricht der Titelverbindung in der Formel (II).

### Beispiel 2.10

Es wurde eine Suspension von Malonsäure der Formel (III) in Acetonitril [1,7 g in 6 mL] hergestellt. Die Suspension wurde 12 h zwischen 20°C und 25°C gerührt. 103 mg Rivaroxaban der Formel (I) in der Modifikation I wurden mit 4 mL der Malonsäuresuspension versetzt und 4,5 Tage bei Raumtemperatur gerührt. Die Kristalle wurden abgesaugt und an der Luft zwischen 20°C und 25°C getrocknet. Die Kristalle wurden Raman-spektroskopisch geprüft und entsprechen der Titelverbindung in der Formel (II).

**Tabelle 1: Differential Scanning Calorimetry und Thermogravimetrie**

| | Rivaroxaban-Malonsäure-Cokristall | Rivaroxaban Modifikation I | Malonsäure |
|---|---|---|---|
| Schmelzpunkt [°C] | 230 | 230 | 135 |
| Umwandlungspunkt [°C] | 170 | - | 85-110 |
| Masseverlust [Gew.-%] | 14 | 0,1 | <0,5 |

**Tabelle 2: Röntgendiffraktometrie**

| | Peakmaxima | |
|---|---|---|
| Rivaroxaban-Malonsäure-Cokristall [2 Theta] | Rivaroxaban Modifikation I [2 Theta] | Malonsäure [2 Theta] |
| 3.5 | 9.0 | 11.3 |
| 11.9 | 12.0 | 17.4 |
| 13.4 | 14.3 | 17.7 |
| 15.8 | 16.5 | 18.7 |
| 18.7 | 17.4 | 23.1 |
| 19.3 | 19.5 | 23.6 |
| 21.1 | 19.9 | 24.1 |
| 21.4 | 21.7 | 24.8 |
| 22.0 | 22.5 | 25.1 |
| 22.4 | 23.4 | 26.6 |
| 24.0 | 24.1 | 27.0 |
| 27.0 | 24.7 | 31.2 |
| 27.5 | 25.6 | 33.0 |
| 28.7 | 26.6 | 33.3 |
| 29.6 | 28.7 | 33.6 |
| 31.0 | 29.4 | 34.1 |
| 32.6 | 30.1 | 35.3 |
| 36.2 | 31.8 | 35.9 |
| | 32.7 | 36.7 |
| | 34.5 | 37.4 |

**Tabelle 3: IR-Spektroskopie**

| | Peakmaxima | |
|---|---|---|
| Rivaroxaban-Malonsäure-Cokristall [cm⁻¹] | Rivaroxaban Modifikation I [cm⁻¹] | Malonsäure [cm⁻¹] |
| 3345 | 3354 | 2990 |
| 3092 | 3074 | 2946 |
| 2887 | 2977 | 2597 |
| 2604 | 2869 | 1701 |
| 1736 | 1736 | 1435 |
| 1629 | 1668 | 1416 |
| 1619 | 1646 | 1397 |
| 1601 | 1606 | 1311 |
| 1553 | 1546 | 1258 |
| 1518 | 1517 | 1216 |
| 1487 | 1486 | 1169 |
| 1469 | 1470 | 1082 |
| 1418 | 1429 | 961 |
| 1400 | 1411 | 919 |
| 1389 | 1374 | 901 |
| 1357 | 1340 | 770 |
| 1329 | 1324 | |
| 1292 | 1308 | |
| 1243 | 1285 | |
| 1228 | 1230 | |
| 1208 | 1219 | |
| 1145 | 1164 | |
| 1125 | 1147 | |
| 1089 | 1120 | |
| 1053 | 1099 | |
| 1025 | 1078 | |
| 995 | 1056 | |
| 967 | 1011 | |
| 933 | 992 | |
| 921 | 947 | |
| 866 | 932 | |
| 816 | 920 | |
| 798 | 891 | |
| 777 | 865 | |
| 746 | 846 | |
| 709 | 829 | |
| | 776 | |
| | 757 | |
| | 745 | |
| | 731 | |
| | 708 | |

**Tabelle 4: Raman-Spektroskopie**

| | Peakmaxima | |
|---|---|---|
| Rivaroxaban-Malonsäure-Cokristall [cm⁻¹] | Rivaroxaban Modifikation I [cm⁻¹] | Malonsäure [cm⁻¹] |
| 3347 | 3074 | 2990 |
| 3091 | 3024 | 2951 |
| 3077 | 2983 | 2611 |
| 2974 | 2944 | 1686 |
| 2889 | 2899 | 1428 |
| 2834 | 2736 | 1402 |
| 2760 | 2566 | 1284 |
| 2719 | 1722 | 1230 |
| 2593 | 1664 | 1179 |
| 1740 | 1638 | 960 |
| 1720 | 1605 | 938 |
| 1626 | 1548 | 921 |
| 1617 | 1512 | 765 |
| 1602 | 1485 | 641 |
| 1557 | 1473 | 601 |
| 1489 | 1445 | 579 |
| 1469 | 1428 | 432 |
| 1451 | 1343 | 407 |
| 1431 | 1325 | 244 |
| 1390 | 1316 | 145 |
| 1354 | 1307 | 121 |
| 1326 | 1300 | 85 |
| 1303 | 1280 | |
| 1292 | 1239 | |
| 1263 | 1232 | |
| 1246 | 1219 | |
| 1231 | 1185 | |
| 1209 | 1164 | |
| 1192 | 1148 | |
| 1152 | 1123 | |
| 1130 | 1098 | |
| 1101 | 1083 | |
| 1093 | 1011 | |
| 1076 | 990 | |
| 1026 | 963 | |
| 998 | 948 | |
| 970 | 922 | |
| 922 | 891 | |
| 902 | 865 | |
| 867 | 814 | |
| 830 | 792 | |
| 797 | 779 | |
| 780 | 756 | |
| 763 | 745 | |
| 746 | 732 | |
| 725 | 709 | |
| 712 | 687 | |
| 688 | 672 | |
| 672 | 642 | |
| 642 | 608 | |
| 613 | 566 | |
| 563 | 548 | |
| 540 | 515 | |
| 514 | 486 | |
| 490 | 438 | |
| 478 | 420 | |
| 440 | 395 | |
| 415 | 344 | |
| 394 | 277 | |
| 350 | 265 | |
| 298 | 247 | |
| 249 | 207 | |
| 158 | 168 | |
| 111 | 156 | |
| 85 | 111 | |
| | 84 | |

**Tabelle 5: FIR-Spektroskopie**

| | Peakmaxima | |
|---|---|---|
| Rivaroxaban-Malonsäure-Cokristall [cm⁻¹] | Rivaroxaban Modifikation I [cm⁻¹] | Malonsäure [cm⁻¹] |
| 490 | 484 | 453 |
| 473 | 475 | 429 |
| 458 | 458 | 244 |
| 443 | 438 | 222 |
| 439 | 418 | 176 |
| 413 | 394 | 123 |
| 400 | 350 | 86 |
| 353 | 303 | |
| 303 | 298 | |
| 292 | 283 | |
| 255 | 272 | |
| 235 | 247 | |
| 202 | 227 | |
| 169 | 209 | |
| 125 | 180 | |
| 94 | 168 | |
| | 138 | |
| | 119 | |
| | 96 | |

**Tabelle 6: NIR-Spektroskopie**

| | Peakmaxima | |
|---|---|---|
| Rivaroxaban-Malonsäure-Cokristall [cm⁻¹] | Rivaroxaban Modifikation I [cm⁻¹] | Malonsäure [cm⁻¹] |
| 8865 | 8837 | 8545 |
| 8479 | 8479 | 7318 |
| 7868 | 7111 | 7199 |
| 7087 | 6568 | 6989 |
| 6557 | 6445 | 5955 |
| 6440 | 6381 | 5824 |
| 6380 | 6006 | 5737 |
| 6050 | 5944 | 4687 |
| 6007 | 5889 | 4370 |
| 5896 | 5833 | 4277 |
| 5812 | 5764 | 4158 |
| 5640 | 5085 | |
| 5529 | 4911 | |
| 5216 | 4881 | |
| 5065 | 4793 | |
| 4906 | 4637 | |
| 4871 | 4483 | |
| 4815 | 4434 | |
| 4774 | 4380 | |
| 4698 | 4302 | |
| 4608 | 4232 | |
| 4412 | 4170 | |
| 4246 | 4145 | |
| 4210 | 4086 | |
| 4143 | 4031 | |
| 4096 | | |

**Tabelle 7: ¹³C-MAS-NMR-Spektrometrie**

| | Peakmaxima | |
|---|---|---|
| Rivaroxaban-Malonsäure-Cokristall [ppm] | Rivaroxaban Modifikation I [ppm] | Malonsäure [ppm] |
| 30 | 36 | 37 |
| 35 | 38 | 42 |
| 38 | 43 | 91 |
| 41 | 45 | 176 |
| 44 | 48 | |
| 49 | 52 | |
| 51 | 56 | |
| 61 | 58 | |
| 62 | 64 | |
| 65 | 70 | |
| 68 | 73 | |
| 69 | 78 | |
| 74 | 120 | |
| 77 | 123 | |
| 118 | 127 | |
| 120 | 129 | |
| 121 | 131 | |
| 123 | 138 | |
| 128 | 141 | |
| 128 | 157 | |
| 137 | 162 | |
| 140 | 167 | |
| 157 | 168 | |
| 162 | 205 | |
| 169 | 207 | |
| 171 | 212 | |
| 204 | 215 | |
| 207 | 216 | |
| 213 | | |
| 221 | | |

**Tabelle 8: Kristallgitter von Rivaroxaban-Malonsäure-Cokristall**

| Kristallsystem | triklin |
|---|---|
| Raumgruppe | P1 |
| Z | 2 |
| Achsenlänge a [Å] | 8,8012(16) |
| Achsenlänge b [Å] | 9,9396(12) |
| Achsenlänge c [Å] | 24,981(3) |
| α [°] | 86,112(9) |
| β [°] | 87,026(11) |
| γ [°] | 76,353(10) |
| berechnete Dichte [g cm⁻³] | 1,531 |

## Patentansprüche

1. Verbindung Rivaroxaban-Malonsäure-Colaistall der Formel (II)

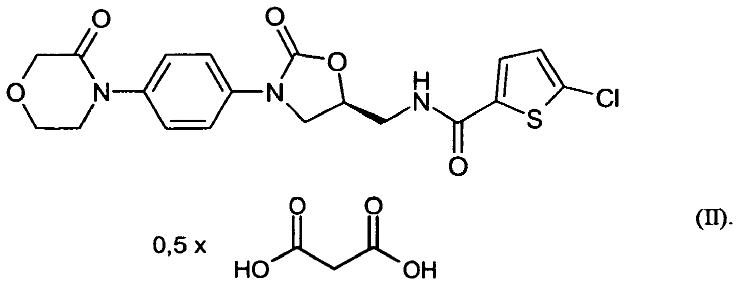

2. Verbindung nach Anspruch 1, **dadurch gekennzeichnet, dass** die Verbindung im Röntgendiffraktogramm einen Reflex bei einem 2-Theta-Winkel von 15,8 aufweist.

3. Verbindung nach Anspruch 1 oder 2, **dadurch gekennzeichnet, dass** die Verbindung im Raman-Spektrum einen Peak bei 1617 cm⁻¹ aufweist.

4. Verfahren zur Herstellung der Verbindung der Formel (II), **dadurch gekennzeichnet, dass** der Wirkstoff Rivaroxaban der Formel (I) 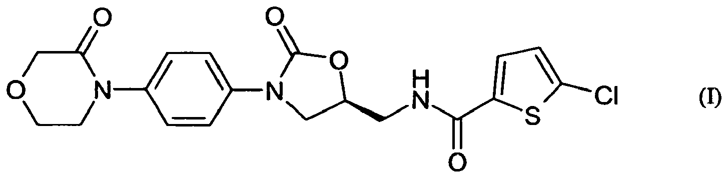 in der Modifikation I und das Reagenz Malonsäure der Formel (III) 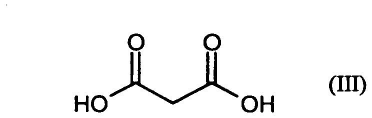 in einem inerten Lösungsmittel bei einer Temperatur zwischen 50°C und 90°C gelöst werden und das Lösungsmittel abgedampft wird.

5. Verfahren nach Anspruch 4, **dadurch gekennzeichnet, dass** der Wirkstoff Rivaroxaban der Formel (I) in der Modifikation I und das Reagenz Malonsäure der Formel (III) im molaren Verhältnis 1:1 eingesetzt werden.

6. Verfahren nach Anspruch 4 oder 5, **dadurch gekennzeichnet, dass** die Lösung vor dem Abdampfen des Lösungsmittels mit Kristallen der Verbindung der Formel (II) angeimpft wird.

7. Verfahren zur Herstellung der Verbindung der Formel (II), **dadurch gekennzeichnet, dass** der Wirkstoff Rivaroxaban der Formel (I) 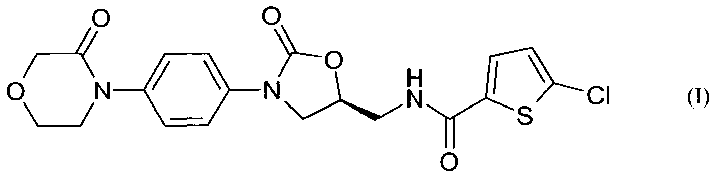 in der Modifikation I und das Reagenz Malonsäure der Formel (III) 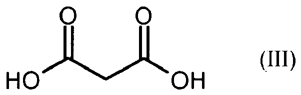 in einem inerten Lösungsmittel bei einer Temperatur zwischen 20°C und 25°C suspendiert werden und der Niederschlag isoliert wird.

8. Verbindung nach einem der Ansprüche 1 bis 3 zur Behandlung und/oder Prophylaxe von Krankheiten.

9. Verwendung der Verbindung nach einem der Ansprüche 1 bis 3 zur Herstellung eines Arzneimittels zur Behandlung und/oder Prophylaxe von thromboembolischen Erkrankungen.

10. Verwendung der Verbindung nach einem der Ansprüche 1 bis 3 zur Verhinderung der Blutkoagulation in vitro.

11. Arzneimittel enthaltend die Verbindung nach einem der Ansprüche 1 bis 3 in Kombination mit einem inerten, nicht-toxischen, pharmazeutisch geeigneten Hilfsstoff.

12. Arzneimittel enthaltend die Verbindung nach einem der Ansprüche 1 bis 3 in Kombination mit einem weiteren Wirkstoff.

13. Arzneimittel nach Anspruch 11 oder 12 zur Behandlung und/oder Prophylaxe von thromboembolischen Erkrankungen.

14. Verfahren zur Verhinderung der Blutkoagulation in vitro, **dadurch gekennzeichnet, dass** eine antikoagulatorisch wirksame Menge der Verbindung nach einem der Ansprüche 1 bis 3 zugegeben wird.

## Claims

1. Compound rivaroxaban/malonic acid cocrystal of the formula (II)

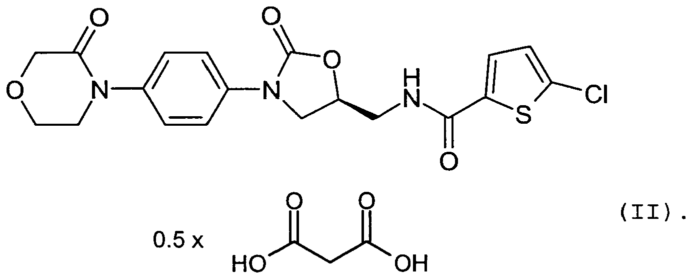

2. Compound according to Claim 1, **characterized in that** the X-ray diffractogram of the compound has a reflex at a 2-theta angle of 15.8.

3. Compound according to Claim 1 or 2, **characterized in that** the Raman spectrum of the compound has a peak at 1617 cm⁻¹.

4. Process for preparing the compound of the formula (II), **characterized in that** the active compound rivaroxaban of the formula (I) 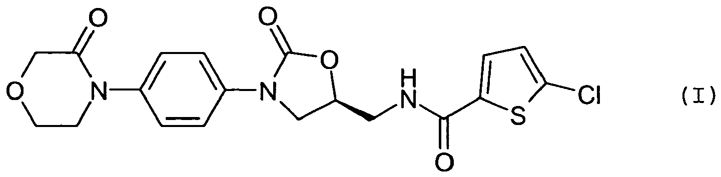 in modification I and the reagent malonic acid of the formula (III) 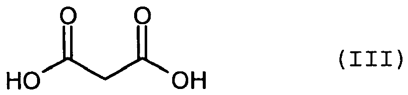 are dissolved in an inert solvent at a temperature between 50°C and 90°C and the solvent is evaporated.

5. Process according to Claim 4, **characterized in that** the active compound rivaroxaban of the formula (I) in modification I and the reagent malonic acid of the formula (III) are employed in a molar ratio of 1:1.

6. Process according to Claim 4 or 5, **characterized in that**, prior to evaporation of the solvent, the solution is seeded with crystals of the compound of the formula (II).

7. Process for preparing the compound of the formula (II), **characterized in that** the active compound rivaroxaban of the formula (I) 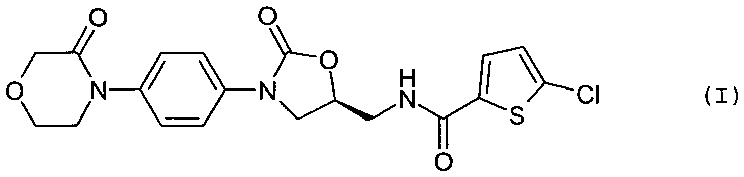 in modification I and the reagent malonic acid of the formula (III) 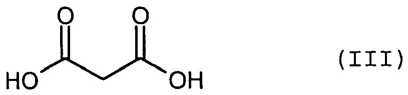 are suspended in an inert solvent at a temperature between 20°C and 25°C and the precipitate is isolated.

8. Compound according to any of Claims 1 to 3 for the treatment and/or prophylaxis of diseases.

9. Use of the compound according to any of Claims 1 to 3 for preparing a medicament for the treatment and/or prophylaxis of thromboembolic disorders.

10. Use of the compound according to any of Claims 1 to 3 for preventing the coagulation of blood in vitro.

11. Medicament, comprising the compound according to any of Claims 1 to 3 in combination with an inert nontoxic pharmaceutically suitable auxiliary.

12. Medicament, comprising the compound according to any of Claims 1 to 3 in combination with a further active compound.

13. Medicament according to Claim 11 or 12 for the treatment and/or prophylaxis of thromboembolic disorders.

14. Method for preventing the coagulation of blood in vitro, **characterized in that** an anticoagulatory amount of the compound according to any of Claims 1 to 3 is added.

## Revendications

1. Composé cocristallin de rivaroxaban et d'acide malonique de formule (II)

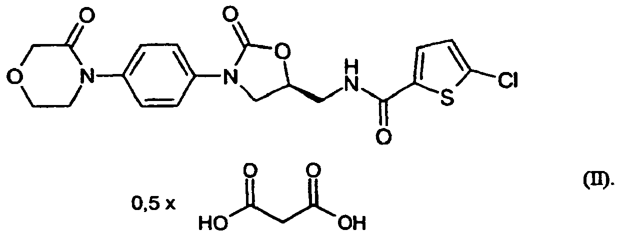

2. Composé selon la revendication 1, **caractérisé en ce que** le composé présente, dans un diffractogramme de rayons X, une réflexion à un angle 2-Thêta de 15,8.

3. Composé selon la revendication 1 ou 2, **caractérisé en ce que** le composé présente, dans un spectre Raman, un pic à 1617 cm⁻¹.

4. Procédé pour la préparation du composé de formule (II), **caractérisé en ce que** la substance active rivaroxaban de formule (I) 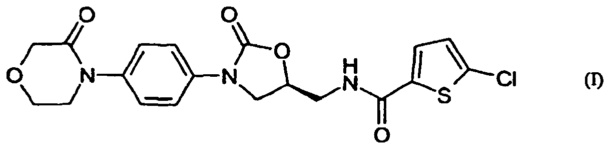 dans la modification I et le réactif acide malonique de formule (III) 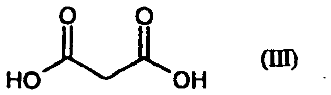 sont dissous dans un solvant inerte à une température entre 50°C et 90°C et le solvant est éliminé par évaporation.

5. Procédé selon la revendication 4, **caractérisé en ce que** la substance active rivaroxaban de formule (I) dans la modification I et le réactif acide malonique de formule (III) sont utilisés dans un rapport molaire de 1:1.

6. Procédé selon la revendication 4 ou 5, **caractérisé en ce que** la solution est ensemencée par des cristaux du composé de formule (II) avant l'élimination par évaporation du solvant.

7. Procédé pour la préparation du composé de formule (II), **caractérisé en ce que** la substance active rivaroxaban de formule (I) 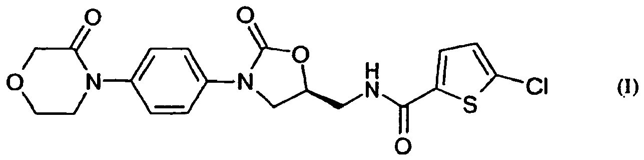 dans la modification I et le réactif acide malonique de formule (III) 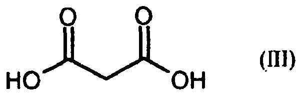 sont mis en suspension dans un solvant inerte à une température entre 20°C et 25°C et le précipité est isolé.

8. Composé selon l'une quelconque des revendications 1 à 3 destiné au traitement et/ou à la prophylaxie de maladies.

9. Utilisation du composé selon l'une quelconque des revendications 1 à 3 pour la préparation d'un médicament destiné au traitement et/ou à la prophylaxie de maladies thromboemboliques.

10. Utilisation du composé selon l'une quelconque des revendications 1 à 3, destinée à empêcher la coagulation du sang in vitro.

11. Médicament contenant le composé selon l'une quelconque des revendications 1 à 3 en combinaison avec un adjuvant inerte, non toxique, pharmaceutiquement approprié.

12. Médicament contenant le composé selon l'une quelconque des revendications 1 à 3 en combinaison avec une autre substance active.

13. Médicament selon la revendication 11 ou 12 destiné au traitement et/ou à la prophylaxie de maladies thromboemboliques.

14. Procédé pour empêcher la coagulation du sang in vitro, **caractérisé en ce qu'**on ajoute une quantité active en anticoagulation du composé selon l'une quelconque des revendications 1 à 3.
